# EUROPEAN PATENT APPLICATION

(11) **EP 4 599 754 A1**
(43) Date of publication of application: **13.08.2025**
(21) Application number: 24382134.5
(22) Date of filing: 12.02.2024
(51) Int. Cl.: A61B 5/00, A61B 10/00, G06T 7/00

(54) **METHOD, SYSTEM AND COMPUTER PROGRAMS FOR CHARACTERIZING THE MENSTRUAL CYCLE PHASES**

(71) Applicant: IVF Spin-Off 1 S.L., 46004 Valencia (ES)
(72) Inventor: Díaz Gimeno, Patricia, 46111 Rocafort (Valencia) (ES); Pellicer Martínez, Antonio, 00197 Roma (IT)
(74) Representative: Carlos Hernando, Borja

(57) **Abstract**

A method, system and computer programs for characterizing the menstrual cycle phases are provided. The method comprises providing/using a database of endometrial spectral images obtained from different females, the images comprising different spectral bands and belonging to an endometrial proliferative phase, to an endometrial early secretory phase, to an endometrial mid-secretory phase and to an endometrial late secretory phase; preprocessing the endometrial spectral images; analyzing differences between the endometrial phases for each wavelength position; and determining wavelength ranges distinguishing the different endometrial phases by comparing the analyzed differences between the endometrial phases, such that the determined wavelength ranges can be used as spectral signature biomarkers for the classification by artificial intelligence techniques of future images with high accuracy.

## Description

### TECHNICAL FIELD

The present invention relates to methods and systems for the characterization of menstrual cycle phases. More specifically, the invention relates to the characterization of the menstrual cycle phases using hyperspectral technology and artificial intelligence algorithms.

### BACKGROUND OF THE INVENTION

One key factor for reproductive success is the status of the maternal endometrium that supports embryo implantation.

The human endometrium is a dynamic tissue that undergoes molecular and morphological modifications throughout the menstrual cycle. Under the control of estrogen and progesterone, these tissue dynamics ultimately promote a state of receptivity that enables embryo implantation. This short period of the mid-secretory phase is called the window of implantation (WOI).

Understanding the WOI is crucial when trying to conceive, as successful implantation is a key factor in achieving pregnancy.

At present, some different methods/techniques are known for endometrial dating to identify the WOI such as histological evaluation, Noyes criteria, biomarkers, ultrasound imaging, Endometrial Receptivity Array (ERA), etc. These methods are often used in combination to provide a more comprehensive assessment of the endometrium and its receptivity during the menstrual cycle. Clinicians may choose the method or combination of methods based on factors such as patient history, fertility treatments, and the need for personalized approaches.

The known methods require an endometrial biopsy, therefore the endometrial status has to be inferred from one cycle to the next (i.e. one menstrual cycle has to be waited for). As a result, the embryo transfer cannot be performed in the same cycle of evaluation. Besides the above, hyperspectral imaging involves capturing and processing information from a large number of spectral bands across the electromagnetic spectrum in a noninvasive manner. Hyperspectral imaging has the potential to provide detailed information about tissue characteristics and molecular composition based on its spectral properties. It can capture a range of wavelengths beyond what the human eye can perceive, allowing for the analysis of subtle variations in tissues.

While hyperspectral imaging has been applied in various fields for detailed spectral analysis, its specific use for endometrial dating and the identification of the WOI is unknown.

New methods and systems for characterizing the menstrual cycle phases using hyperspectral technology are therefore needed.

### DESCRIPTION OF THE INVENTION

The object of present invention is to distinguish the endometrial cyclic changes to be able to characterize the WOI in depth using hyperspectral technology. This characterization could serve as a diagnostic tool, enabling the determination of the optimal moment for the implantation of an embryo in a woman's uterus in a less invasive and more accurate way, thus minimizing the time to pregnancy and improving the woman's quality of life. Likewise, the proposed method advantageously allows embryo transfer in the same evaluation cycle, without inference from one cycle to the following cycle.

To that end, the present invention proposes, according to one aspect, a computer implemented method comprising providing/using a database of endometrial spectral images obtained from different females, the database storing one or more endometrial images for each female, and the endometrial spectral images comprising different spectral bands in a range between 400 and 1000 nanometers and comprising images belonging to an endometrial proliferative phase, to an endometrial early secretory phase, to an endometrial mid-secretory phase and to an endometrial late secretory phase; preprocessing the endometrial spectral images; analyzing differences between the endometrial phases for each wavelength position using classical statistical techniques and/or artificial intelligence techniques; and determining wavelength ranges that distinguish the different endometrial phases by comparing the analyzed differences between the endometrial phases.

Present invention also proposes, according to another aspect, a system for characterizing the menstrual cycle phases, comprising:
a database to store endometrial spectral images from different females, the database being configured to store one or more endometrial images for each female, and the endometrial spectral images comprising different spectral bands in a range between 400 and 1000 nanometers and comprising images belonging to an endometrial proliferative phase, to an endometrial early secretory phase, to an endometrial mid-secretory phase and to an endometrial late secretory phase;
one or more processors configured to preprocess the endometrial spectral images; analyze differences between the endometrial phases for each wavelength position using classical statistical techniques and/or artificial intelligence techniques; and determine wavelength ranges that distinguish the different endometrial phases by comparing the analyzed differences between the endometrial phases.

Consequently, the determined wavelength ranges can be used as spectral signature biomarkers for the classification of future endometrial spectral images with high accuracy.

In some embodiments, the determined wavelength ranges comprise 430nm-456nm and 584nm-630nm for the endometrial proliferative phase vs the endometrial early secretory phase; 428nm-442nm, 458nm-490nm, 504nm, 516nm, 548nm-552nm, 566nm-576nm, and 984nm-988nm for the endometrial proliferative phase vs the endometrial mid-secretory phase; 426nm-442nm, 464nm, 478nm-486nm, and 588nm-610nm for the endometrial proliferative phase vs the endometrial late secretory phase; 440nm-464nm, 472nm-476nm, 498nm-502nm, and 528nm-636nm for the endometrial early secretory phase vs the endometrial mid-secretory phase; 414nm-416nm, 450nm-508nm, 526nm-528nm, 586nm, 608nm-664nm, 672nm-696nm, 778nm-788nm, 800nm-806nm, and 846nm-876nm for the endometrial early secretory phase vs the endometrial late secretory phase; 414nm-416nm, 450nm-508nm, 526nm-528nm, 586nm, 608nm-664nm, 672nm-696nm, 778nm-806nm, 846nm-850nm, and 874nm-876nm for the endometrial mid-secretory phase vs. the endometrial late secretory phase. Therefore, these wavelength ranges can be distinctly used for characterizing the different phases of the menstrual cycle, with a specific focus on the mid secretory phase.

In some embodiments, the preprocessing of the endometrial spectral images comprises checking whether the endometrial spectral images fulfill one or more evaluation criteria; extracting the spectra of the endometrial spectral images that fulfill the one or more evaluation criteria using a computed mask, and normalizing the extracted spectra per area, obtaining a normalized spectra for each endometrial phase as a result; and calculating mean spectra for each of the endometrial phases using the obtained normalized spectra.

The evaluation criteria can comprise i) a shape factor that ensures that an image waveform is similar to a characteristic waveform of the endometrium, ii) an intensity factor that ensures that an intensity of the spectral bands exceeds a given intensity threshold range, and/or iii) a signal to noise ratio that ensures that a signal to noise ratio in the 800-900nm band does not exceed a given noise threshold value.

In some embodiments, the mask is computed using Otsu's thresholding method combined with the erosion technique.

In some embodiments, the classical statistical techniques comprise an Analysis Of Variance, ANOVA, test or a t-test.

In some embodiments, the artificial intelligence techniques comprise Machine Learning algorithms such as SVM, Random Forest, KNN, etc., Deep Learning algorithms such as CNNs, LSTMs, etc., and/or one or more neural networks.

In some embodiments, the endometrial spectral images are acquired using a multispectral camera that is operably connected to a fiberscope, the latter being configured to be introduced inside a transfer cannula of a given diameter.

Other embodiments of the invention that are disclosed herein also include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

### BRIEF DESCRIPTION OF THE DRAWINGS

The previous and other advantages and features will be more fully understood from the following detailed description of embodiments, with reference to the attached figures, which must be considered in an illustrative and non-limiting manner, in which:
Fig. 1 schematically shows the steps used for characterizing the menstrual cycle phases, particularly, the mid secretory phase where embryo implantation occurs, according to an embodiment.
Figs. 2A-2C schematically show an embodiment of the setup used for the characterization of the menstrual cycle phases.
Fig. 3A is a graph of the spectra obtained from one image. Fig. 3B is a plot of the average of all the spectra of an image.
Fig. 4 is a graph illustrating the wavelength positions with higher differences obtained by means of the ANOVA test.
Fig. 5 is a graph illustrating the wavelength positions with higher differences obtained by means of the t-test.

### DETAILED DESCRIPTION OF THE INVENTION AND OF PREFERRED EMBODIMENTS

To provide an overall understanding of the invention, certain illustrative embodiments will now be described, including a method and a system for characterizing the menstrual cycle phases. However, it will be understood by one of ordinary skill in the art that the method and system described herein may be adapted and modified as is appropriate for the application being addressed.

The present invention facilitates the identification of wavelength ranges that distinctly characterize different endometrial phases, including the endometrial proliferative phase (PF), endometrial early secretory phase (ESE), endometrial mid-secretory phase (MSE), and endometrial late secretory phase (LSE). The defined wavelength ranges serve as spectral signature biomarkers. That is, a characterization of the hyperspectral signature of the human endometrium is provided.

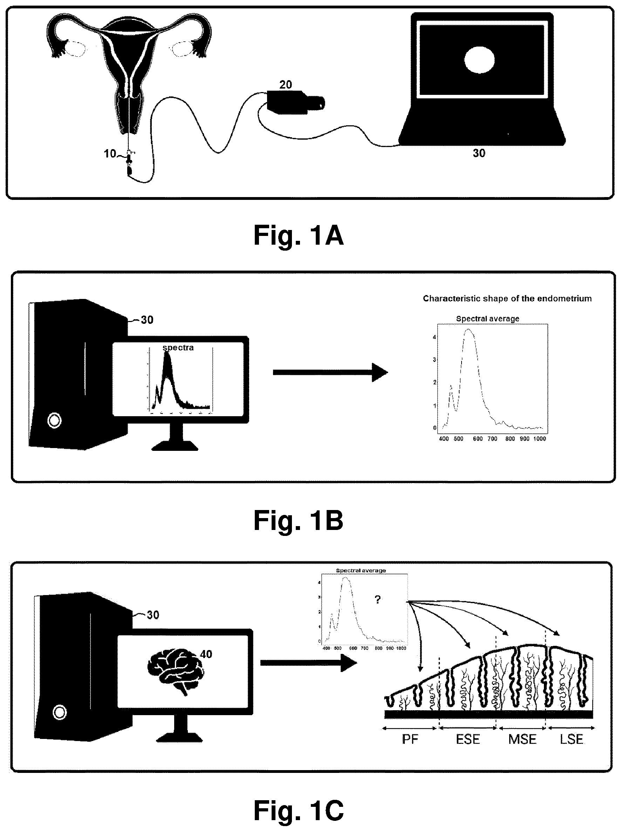

Referring to Figs. 1A-1C, a schematic of the proposed architecture is shown encompassing a sample collection phase, a data analysis phase, and a sample classification phase in each endometrial phase. During the sample collection phase, a micro-fiberscope 10 is employed to capture endometrial spectral images with a hyperspectral camera 20. Particularly, the micro-fiberscope is introduced in an embryo transfer catheter, reaching the fundus of the uterine cavity. The hyperspectral camera 20 in some embodiments can acquire 300 spectral bands in the range between 400 and 1000 nm. The captured images are processed with a computing device 30, such as a PC a notebook, or the like, running specialized software to obtain the endometrial spectra.

In the data analysis phase, data preprocessing/analysis of the endometrial spectra is conducted on the same computing device 30 or another suitable device, to obtain the characteristic shape of the endometrium. This can be done by normalizing the different endometrial spectra by area. As can be seen, the intensity of the characteristic shape of the endometrium is above 1000 nm - 1500 nm. There is also a first peak between 400 nm and 500 nm below the first intensity quartile, and a second peak between 500 nm and 700 nm above the third intensity quartile. Between 800 nm and 900 nm the intensity should not exceed 30% of the maximum signal value.

In the subsequent sample classification phase, a signature discovery process is done to select the optimal wavelength range for identifying the WOI. Various classification models 40, such as Support Vector Machine (SVM) algorithms, Deep Learning algorithms, and/or neural networks may be employed to predict the endometrial phase in which the endometrial spectra is present. K-fold cross-validation techniques for ensuring the signature discovery performance avoiding overfitting and providing population inference can be also used. In some embodiments, to evaluate the classification models 40, some prediction parameters thereof such as accuracy, sensitivity, and specificity are computed.

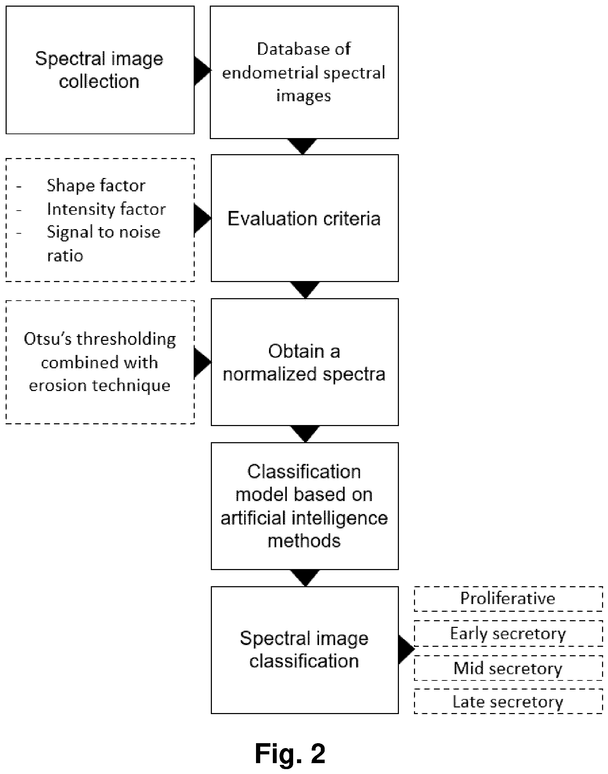

The invention, in one embodiment, is generally directed to a computer implemented method for characterizing the menstrual cycle phases in a non-invasive way using hyperspectral technology. Fig. 2 shows an embodiment of the proposed method, where the execution involves the use of endometrial spectral images, from different females, stored in a database or a similar repository. These spectral images can be obtained through the aforementioned sample collection phase and encompass various spectral bands within the 400-1000 nm range. They may correspond to the PF, ESE, MSE, or LSE phase.

According to this embodiment, the method, as part of the images' preprocessing, performs an evaluation criteria on the collected endometrial spectral images or at least a portion of them, to assess their suitability. The evaluation criteria may involve checking various factors, including checking a shape factor to ensure that an image waveform is similar to the characteristic waveform of the endometrium, checking an intensity factor to ensure that the intensity of the spectral bands exceeds a given intensity threshold range, and/or checking a signal to noise ratio to ensure that the signal to noise ratio in the 800-900nm band does not exceed a given noise threshold value.

Once the evaluation criteria is executed, the method extracts the spectra of the endometrial spectral images that meet the criteria. Subsequently, normalized spectra are obtained for each endometrial phase.

In certain embodiments, the extraction of valid spectra is automated using a dynamically calculated mask for each image. The Otsu thresholding method, in conjunction with the erosion technique, can be employed to calculate this mask. This method aids in identifying the area of the endometrium with the highest light intensity in the image.

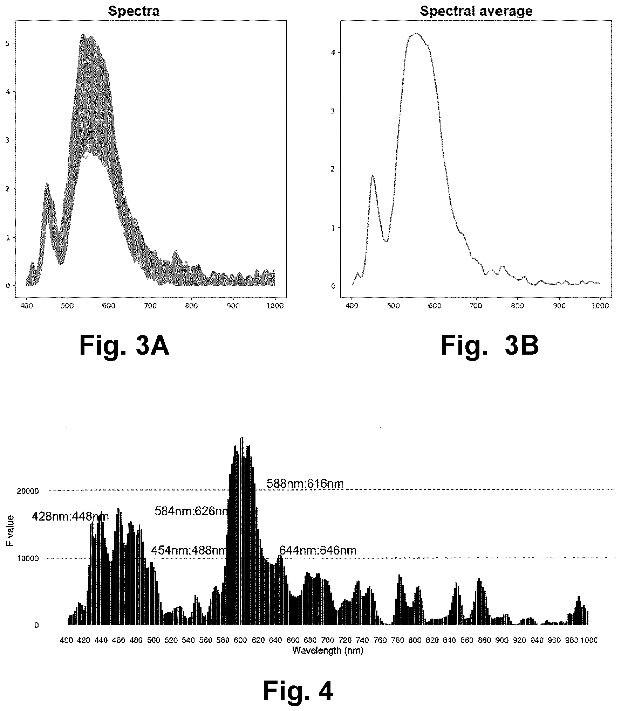

The method proceeds with the final step of the image's preprocessing, which particularly involves the calculation of the mean spectra for each of the endometrial phases (as illustrated in Fig. 3A and 3B).

Subsequently, differences between the endometrial phases are analyzed at each wavelength position. This analysis can be conducted using classical statistical techniques such as the ANOVA test or the t-test. Alternatively or complementarily, artificial intelligence techniques can be employed for a more sophisticated analysis of spectral variations among the different endometrial phases.

In the final step, the method performs the spectral image classification to identify the wavelength ranges that effectively distinguish between the various endometrial phases.

Fig. 4 shows the wavelength positions with higher differences obtained by means of the ANOVA test.

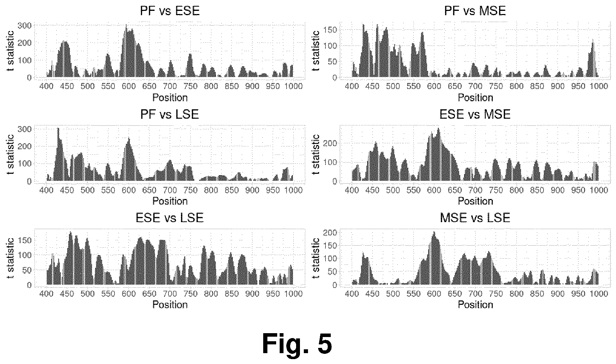

Similarly, Fig. 5 displays the wavelength positions with higher differences, as identified through the t-test. In this case, particularly, the wavelength differences between the endometrial phases can be summarized as follows:
- PF vs ESE: 430nm-456nm, 584nm-630nm
- PF vs MSE: 428nm-442nm, 458nm-490nm, 504nm, 516nm, 548nm-552nm, 566nm-576nm, and 984nm-988nm
- PF vs LSE: 426nm-442nm, 464nm, 478nm-486nm, and 588nm-610nm
- ESE vs MSE: 440nm-464nm, 472nm-476nm, 498nm-502nm, and 528nm-636nm
- ESE vs LSE: 414nm-416nm, 450nm-508nm, 526nm-528nm, 586nm, 608nm-664nm, 672nm-696nm, 778nm-788nm, 800nm-806nm, and 846nm-876nm
- MSE vs LSE: 414nm-416nm, 450nm-508nm, 526nm-528nm, 586nm, 608nm-664nm, 672nm-696nm, 778nm-806nm, 846nm-850nm, and 874nm-876nm.

The system and/or functionality of the invention may be implemented via various combinations of software and hardware, as described, or entirely in software elements. Also, particular divisions of functionality between the various components described herein are merely exemplary, and not mandatory or significant. Consequently, functions performed by a single component may, in other embodiments, be performed by multiple components, and functions performed by multiple components may, in other embodiments, be performed by a single component.

Certain aspects of the present invention include process steps or operations and instructions described herein in an algorithmic and/or algorithmic-like form. It should be noted that the process steps and/or operations and instructions of the present invention can be embodied in software, firmware, and/or hardware, and when embodied in software, can be downloaded to reside on and be operated from different platforms used by real-time network operating systems.

The scope of the present invention is defined in the following set of claims.

## Claims

1. A computer implemented method for characterizing the menstrual cycle phases, comprising:
providing or using a database of endometrial spectral images obtained from different females, the database storing one or more endometrial images for each female, and the endometrial spectral images comprising different spectral bands in a range between 400 and 1000 nanometers and comprising images belonging to an endometrial proliferative phase, to an endometrial early secretory phase, to an endometrial mid-secretory phase and to an endometrial late secretory phase;
preprocessing the endometrial spectral images;
analyzing differences between the endometrial phases for each wavelength position using classical statistical techniques and/or artificial intelligence techniques; and
determining wavelength ranges that distinguish the different endometrial phases by comparing the analyzed differences between the endometrial phases,
such that the determined wavelength ranges can be used as spectral signature biomarkers for the classification of future endometrial spectral images.

2. The method of claim 1, wherein the determined wavelength ranges comprise:
430nm-456nm and 584nm-630nm for the endometrial proliferative phase vs the endometrial early secretory phase;
428nm-442nm, 458nm-490nm, 504nm, 516nm, 548nm-552nm, 566nm-576nm, and 984nm-988nm for the endometrial proliferative phase vs the endometrial mid-secretory phase;
426nm-442nm, 464nm, 478nm-486nm, and 588nm-610nm for the endometrial proliferative phase vs the endometrial late secretory phase;
440nm-464nm, 472nm-476nm, 498nm-502nm, and 528nm-636nm for the endometrial early secretory phase vs the endometrial mid-secretory phase;
414nm-416nm, 450nm-508nm, 526nm-528nm, 586nm, 608nm-664nm, 672nm-696nm, 778nm-788nm, 800nm-806nm, and 846nm-876nm for the endometrial early secretory phase vs the endometrial late secretory phase;
414nm-416nm, 450nm-508nm, 526nm-528nm, 586nm, 608nm-664nm, 672nm-696nm, 778nm-806nm, 846nm-850nm, and 874nm-876nm for the endometrial mid-secretory phase vs. the endometrial late secretory phase.

3. The method of claim 1, wherein the preprocessing of the endometrial spectral images comprises:
checking whether the endometrial spectral images fulfill one or more evaluation criteria;
extracting the spectra of the endometrial spectral images that fulfill the one or more evaluation criteria using a computed mask, and normalizing the extracted spectra per area, obtaining a normalized spectra for each endometrial phase as a result; and
calculating mean spectra for each of the endometrial phases using the obtained normalized spectra.

4. The method of claim 3, wherein the evaluation criteria comprise:
a shape factor that ensures that an image waveform is similar to a characteristic waveform of the endometrium, and/or
an intensity factor that ensures that an intensity of the spectral bands exceeds a given intensity threshold range, and/or
a signal to noise ratio that ensures that a signal to noise ratio in the 800-900nm band does not exceed a given noise threshold value.

5. The method of any one of the previous claims, wherein the mask is computed using Otsu's thresholding method combined with the erosion technique.

6. The method of any one of the previous claims, wherein the classical statistical techniques comprise an ANalysis Of VAriance, ANOVA, test or a t-test.

7. The method of any one or the previous claims, wherein the artificial intelligence techniques comprise a Machine Learning algorithm, a Deep Learning algorithm, one or more neural networks and/or the combination thereof.

8. A system for characterizing the menstrual cycle phases, comprising:
a database configured to store endometrial spectral images from different females, the database being configured to store one or more endometrial images for each female, and the endometrial spectral images comprising different spectral bands in a range between 400 and 1000 nanometers and comprising images belonging to an endometrial proliferative phase, to an endometrial early secretory phase, to an endometrial mid-secretory phase and to an endometrial late secretory phase;
one or more processors configured to:
preprocess the endometrial spectral images;
analyze differences between the endometrial phases for each wavelength position using classical statistical techniques or machine learning techniques; and
determine wavelength ranges that distinguish the different endometrial phases by comparing the analyzed differences between the endometrial phases,
such that the determined wavelength ranges can be used as spectral signature biomarkers for the classification of future endometrial spectral images.

9. The system of claim 8, wherein the endometrial spectral images are acquired using a multispectral camera (20) that is operably connected to a fiberscope (10), the latter being configured to be introduced inside a transfer cannula of a given diameter.

10. A non-transitory computer readable medium comprising code instructions that when executed by a computing device implement the method of any of claims 1 to 8.
